# EUROPEAN PATENT APPLICATION

(11) **EP 2 682 142 A1**
(43) Date of publication of application: **08.01.2014**
(21) Application number: 12175346.1
(22) Date of filing: 06.07.2012
(51) Int. Cl.: A61M 5/20, A61M 5/24, A61M 5/00

(54) **Autoinjector**

(71) Applicant: Sanofi-Aventis Deutschland GmbH, 65929 Frankfurt am Main (DE)
(72) Inventor: The designation of the inventor has not yet been filed

(57) **Abstract**

Described is an autoinjector (1) comprising a case (2) having a hole, a carrier adapted to hold a syringe (4) and slidably disposed in the case (2), a cover (8) telescopically coupled to the case (2) and translatable relative to the case (2) between a first position and a second position in which the cover (8) covers the hole, and a releasable locking mechanism adapted to lock the cover (8) in the second position.

## Description

### Technical Field

The invention relates to an autoinjector for administering a medicament.

### Background of the Invention

Administering an injection is a process which presents a number of risks and challenges for users and healthcare professionals, both mental and physical. Injection devices typically fall into two categories - manual devices and autoinjectors. In a conventional manual device, manual force is required to drive a medicament through a needle. This is typically done by some form of button / plunger that has to be continuously pressed during the injection. There are numerous disadvantages associated with this approach. For example, if the button / plunger is released prematurely, the injection will stop and may not deliver an intended dose. Further, the force required to push the button /plunger may be too high (e.g., if the user is elderly or a child). And, aligning the injection device, administering the injection and keeping the injection device still during the injection may require dexterity which some patients (e.g., elderly patients, children, arthritic patients, etc.) may not have.

Autoinjector devices aim to make self-injection easier for patients. A conventional autoinjector may provide the force for administering the injection by a spring, and trigger button or other mechanism may be used to activate the injection. Autoinjectors may be single-use or reusable devices.

Autoinjectors may be mechanical, electro-mechanical or fully electronic. Conventional mechanical autoinjectors may automatically provide the required force for needle insertion and medicament delivery, but may not provide additional functionality (e.g., alignment verification, injection site verification, etc.) which may be capable with electro-mechanical and fully electronic autoinjectors.

Thus, there remains a need for an improved autoinjector.

### Summary of the Invention

It is an object of the present invention to provide an improved autoinjector.

In an exemplary embodiment, an autoinjector according to the present invention comprises a case having a hole, a carrier adapted to hold a syringe and slidably disposed in the case, a cover telescopically coupled to the case and translatable relative to the case between a first position and a second position in which the cover covers the hole, and a releasable locking mechanism adapted to lock the cover in the second position.

In an exemplary embodiment, the autoinjector further comprises an energy source including a disposable battery or a rechargeable battery.

In an exemplary embodiment, the autoinjector further comprises a plunger adapted to advance a stopper in the syringe, a motor, and a gear train operably coupled to the motor. The gear train includes a pinion adapted to actuate a rack disposed on the plunger.

In an exemplary embodiment, the autoinjector further comprises a controller operably coupled to the motor.

In an exemplary embodiment, the autoinjector further comprises a depressible button coupled to the case. The button includes a transparent cap and one or more light emitting elements. The one or more light emitting elements includes a plurality of light emitting elements, and each of the light emitting elements is adapted to emit a different color light.

In an exemplary embodiment, the autoinjector further comprises a first sensor adapted to generate a first signal when the cover is locked in the second position.

In an exemplary embodiment, the autoinjector further comprises a second sensor adapted to generate a second signal when the autoinjector is placed on an injection site.

In an exemplary embodiment, the autoinjector further comprises a third sensor adapted to generate a third signal based on a position of the carrier or the plunger. The third sensor includes an encoder wheel and an optoelectronic coupler.

In an exemplary embodiment, the autoinjector further comprises a cap releasably coupled to the case and having a grip adapted to releasably engage a needle boot.

In an exemplary embodiment, the autoinjector further comprises a fourth sensor adapted to generate a fourth signal when the cap is coupled to the case. The controller changes a color or illumination sequence of the one or more light emitting elements based on the first signal, the second signal, the third signal or the fourth signal. The controller operates the releasable locking mechanism based on the first signal, the second signal, the third signal or the fourth signal.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(O2)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2, H-Asn-(Glu)5-des Pro36, Pro37, Pro38 [Trp(02)25, Asp28] Exendin-4(1-39)-(Lys)6-NH2, H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2, H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
Figure 1 is a schematic front view of an exemplary embodiment of an autoinjector according to the present invention,
Figure 2 is a schematic lateral view an exemplary embodiment of an autoinjector according to the present invention,
Figure 3 is a schematic rear view an exemplary embodiment of an autoinjector according to the present invention,
Figure 4 is an exemplary embodiment of a method for initializing an autoinjector according to the present invention, and
Figure 5 is an exemplary embodiment of a method for performing an injection with an autoinjector according to the present invention.

Corresponding parts are marked with the same reference symbols in all figures.

### Detailed Description

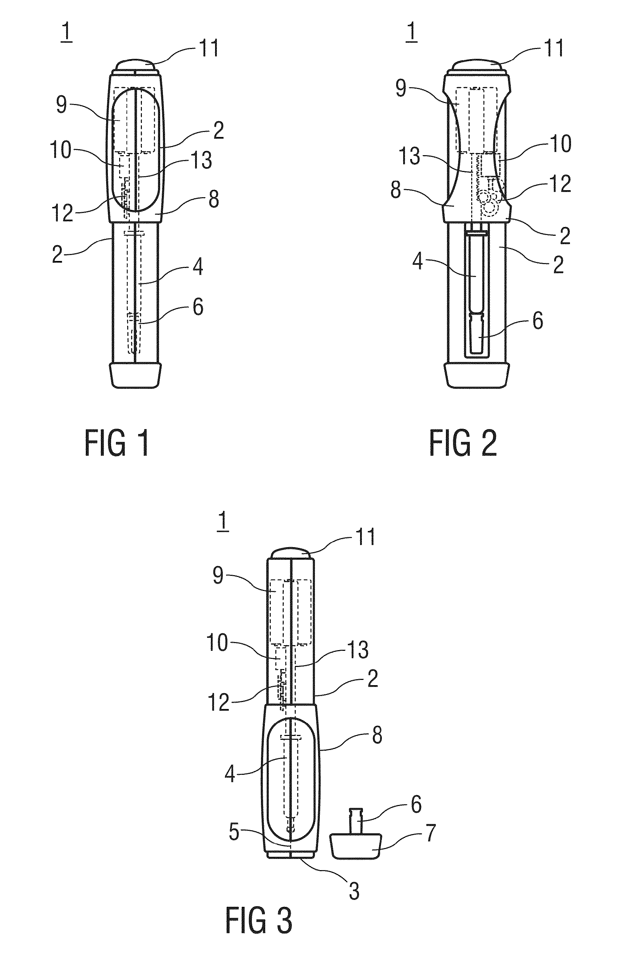

Figure 1 is a schematic front view of an exemplary embodiment of an autoinjector 1 according to the present invention. Figure 2 is a corresponding lateral view and figure 3 a corresponding rear view of an exemplary embodiment of an autoinjector 1 according to the present invention.

In an exemplary embodiment, the autoinjector 1 comprises a case 2 having an elongate shape with a distal end 3 and a proximal end. The case 2 includes a carrier slidably disposed therein which is adapted to replaceably retain a syringe 4, e.g. a pre-filled syringe with a dose of a medicament. A needle 5 is coupled to a distal end of the syringe 4. A protective needle boot 6 is replacabely coupled to the needle 5.

In an exemplary embodiment, the autoinjector 1 includes a cover 8 slidably disposed on the case 2. The cover 8 is translatable relative to the case 2 between a first, proximal position (shown in Figures 1 and 2) and a second, distal position (shown in Figure 3). When the cover 8 is in the proximal position, the carrier may be exposed via a hole in the case 2, allowing an unused syringe 4 to be inserted into the carrier and a used syringe 4 to be removed from the carrier. When the cover 8 is in the distal position, the cover 8 may enclose the carrier to prevent movement of the syringe 4 relative to a longitudinal axis of the autoinjector 1 but allow for axial translation of the carrier. In an exemplary embodiment, the autoinjector 1 includes a releaseable locking mechanism that engages the cover 8 when it is in the distal position. The locking mechanism may include resilient arms disposed on the autoinjector 1 which engage the cover 8 when it is in the distal position. The arms may be released by manual force (e.g., pulling the cover 8 in the proximal direction, pressing a release button, etc.) or by a motor 10 (e.g., after an injection procedure is complete).

In an exemplary embodiment, the autoinjector 1 includes an energy source 9 (e.g., a disposable or rechargeable battery) and a controller. The autoinjector 1 may further comprise a connector adapted to create a power coupling between the energy source 9 and an external energy source (e.g., for recharging).

In an exemplary embodiment, the autoinjector 1 includes a motor 10 for performing motions related to the injection, e.g., needle insertion, plunger/stopper displacement for medicament delivery, and/or needle retraction. For example, the motor 10 may actuate a gear train 12 to advance the carrier (and the syringe 4 therein) for needle insertion and advance a plunger 13 to push a stopper in the syringe 4 to dispense the medicament therein. As understood by those of skill in the art, the gear train 12 may reduce output speed of the motor 10 and increase its torque in order to deliver the required motions and forces. In an exemplary embodiment, the gear train 12 includes the plunger 13 having a rack and a pinion adapted to engage the rack. The motor 10 may be, for example, a single, compact, high speed brushed DC motor.

In an exemplary embodiment, the motor 10 may actuate the gear train 12 to retract the carrier. In another exemplary embodiment, the autoinjector 1 may include a retraction spring (not shown) disposed between the distal end 3 of the case 2 and the carrier. After an injection when the carrier has compressed the retraction spring against the case 2, the motor 10 may release the force on the gear train 12, allowing the retraction spring to expand and retract the needle 5.

In an exemplary embodiment, the autoinjector 1 includes a control button 11. In an exemplary embodiment, the control button 11 has a transparent cap which is illuminated in a plurality of colors by one or more light emitting elements. Different colors and/or sequences of colors may provide corresponding visual feedback (e.g., green light means the autoinjector 1 is ready for use, red light means the autoinjector 1 is in use, etc.).

In an exemplary embodiment, the autoinjector 1 may include one or more sensors (e.g., mechanical, electronic, optical, etc.). A first sensor may indicate whether the cover 8 is locked in the distal position. For example, the first sensor may generate a first signal when the cover 8 is locked in the distal position. The first signal may be utilized by the controller to control illumination of the light emitting elements and/or operation of the motor 10. A second sensor may indicate whether the autoinjector 1 has been placed on the injection site. For example, the second sensor may generate a second signal when the distal end 3 of the autoinjector 1 is placed on the injection site. The second signal may be utilized by the controller to control illumination of the light emitting elements, operation of the motor 10, and/or operation of the locking mechanism for the cover 8. A third sensor may indicate a position of the carrier and/or the plunger 13. For example, the third sensor may be a slotted encoder wheel in the gear train 12 with an optoelectronic coupler whose optical path may be interrupted by the encoder wheel. The third sensor may generate a third signal which is used by the controller to control illumination of the light emitting elements, operation of the motor 10, and/or operation of the locking mechanism for the cover 8.

In an exemplary embodiment, the autoinjector 1 includes a cap 7 removably coupled to the distal end 3. The cap 7 may include a grip adapted to engage the needle boot 6. In an exemplary embodiment, the cap 7 is operably coupled to the locking mechanism for the cover 8 such that the locking mechanism cannot be released until the cap 7 is coupled to the distal end 3 of the autoinjector 1. A fourth sensor may be included to generate a signal indicating whether the cap 7 is coupled to the autoinjector 1. The fourth sensor may generate a fourth signal which is used by the controller control illumination of the light emitting elements, operation of the motor 10, and/or operation of the locking mechanism for the cover 8.

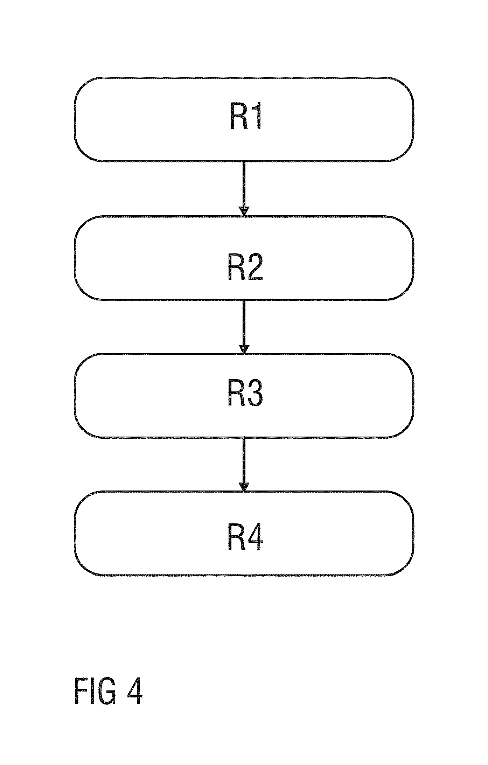

Figure 4 is an exemplary embodiment of a method for initializing an autoinjector according to the present invention. The initialization process may occur after an injection when a used syringe 4 is removed from the autoinjector 1 and replaced with an unused syringe 4. If the autoinjector is delivered to a user with an unused syringe 4, the initialization process may be bypassed.

In step R1, the cap 7 is coupled to the autoinjector 1. The grip of the cap 7 may include the needle boot 6, and coupling the cap 7 to the autoinjector 1 may replace the needle boot 6 on the needle 5. When the cap 7 is coupled to the autoinjector, the locking mechanism for the cover 8 may be released. For example, the fourth sensor may generate the fourth signal which the controller utilizes to release the locking mechanism, which may be sensed by the first sensor. When the locking mechanism is released, the controller may change the illumination of the light emitting element(s) of the button 11 to a different color to indicate that the cover 8 is unlocked and the autoinjector 1 cannot be used.

In step R2, the cover 8 is translated from the distal position to the proximal position, exposing the used syringe 4. In an exemplary embodiment, as the cover 8 is translated from the distal position to the proximal position, a pivotable arm underlying the syringe 4 may rotate and push a portion (e.g., proximal portion) radially away from a longitudinal axis of the autoinjector 1. The used syringe 4 (with the needle boot 6 covering the needle 5) may be easier to remove in this manner.

In step R3, after the used syringe 4 is removed, an unused syringe 4 may be placed in the carrier.

In step R4, the cover 8 is translated from the proximal position to the distal position, and the locking mechanism for the cover 8 is engaged. The first sensor generates the first signal to indicate that the cover 8 is locked in the distal position. The controller may active the motor 10 to advance the carrier a small axial distance so that the grip of the cap 7 engages the needle boot 6.

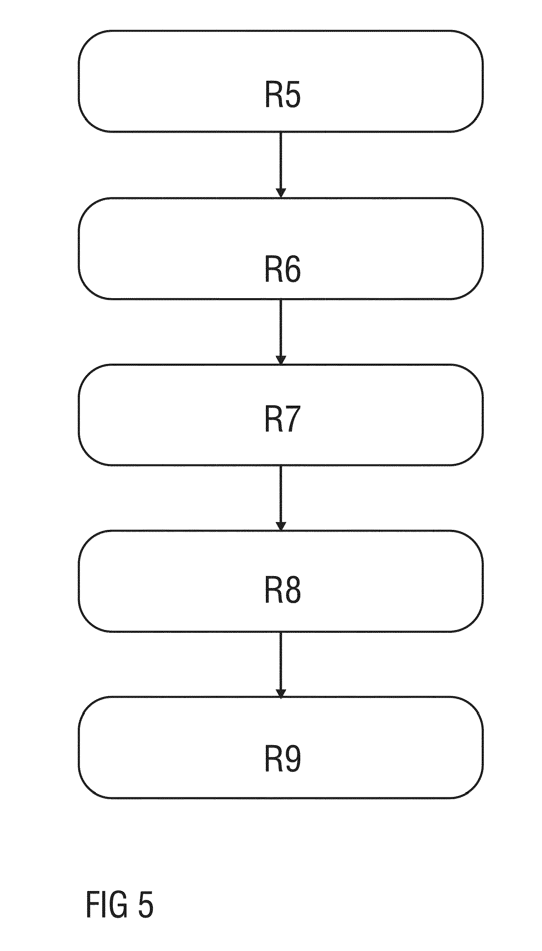

Figure 5 shows an exemplary embodiment of a method for performing an injection with an autoinjector according to the present invention.

In step R5, the cap 7 is removed, which removes the needle boot 6. When the cap 7 is removed, the fourth sensor may generate the fourth signal. However, the controller may not operate the motor 10, because the second sensor has not generated the second signal which indicates that the autoinjector 1 has been placed on the injection site.

In step R6, the autoinjector 1 is placed on an injection site. The second sensor generates the second signal, which the controller may utilize to change an illumination of the light emitting element of the button 11. The illumination change may provide visual notice to the user that the autoinjector 1 is ready for use.

In step R7, when the button 11 is pressed, the controller activates the motor 10 to advance the carrier and the syringe 4 for insertion of the needle 5 into the injection site. In an exemplary embodiment, the controller may change an illumination of the light emitting element of the button 11 to provide visual notice to the user that the injection is being performing. After needle insertion, the controller advances the plunger 13 to push the stopper of the syringe 4 to deliver the medicament.

In step R8, the force of the motor 10 is released and the retraction spring pushes the carrier proximally, withdrawing the needle 5 from the injection site. In another exemplary embodiment, the motor 10 may rotate in reverse to retract the carrier.

In step R9, after the autoinjector 1 has been removed from the injection site, the cap 7 is replaced on the autoinjector 1. When the cap 7 is replaced, the fourth sensor may generate the fourth signal. The controller may change an illumination of the light emitting element of the button 11. The illumination change may provide visual notice to the user that the autoinjector 1 contains a used syringe 4.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. An autoinjector (1) comprising:
a case (2) having a hole;
a carrier adapted to hold a syringe (4) and slidably disposed in the case (2);
a cover (8) telescopically coupled to the case (2) and translatable relative to the case (2) between a first position and a second position, wherein in the second position, the cover (8) covers the hole; and
a releasable locking mechanism adapted to lock the cover (8) in the second position.

2. The autoinjector (1) according to claim 1, further comprising:
an energy source (9) including a disposable battery or a rechargeable battery.

3. The autoinjector (1) according to any one of the preceding claims, further comprising:
a plunger (13) adapted to advance a stopper in the syringe (4);
a motor (10); and
a gear train (12) operably coupled to the motor (10), the gear train (12) including a pinion adapted to actuate a rack disposed on the plunger (13).

4. The autoinjector (1) according to claim 3, further comprising:
a controller operably coupled to the motor (10).

5. The autoinjector (1) according to any of the preceding claims, further comprising:
a depressible button (11) coupled to the case (2).

6. The autoinjector (1) according to claim 5, wherein the button (11) includes a transparent cap and one or more light emitting elements.

7. The autoinjector (1) according to claim 6, wherein the one or more light emitting elements includes a plurality of light emitting elements, each of the light emitting elements adapted to emit a different color light.

8. The autoinjector (1) according to any of the preceding claims, further comprising:
a first sensor adapted to generate a first signal when the cover (8) is locked in the second position.

9. The autoinjector (1) according to any of the preceding claims, further comprising:
a second sensor adapted to generate a second signal when the autoinjector (1) is placed on an injection site.

10. The autoinjector (1) according to claim 3 or 4, further comprising:
a third sensor adapted to generate a third signal based on a position of the carrier or the plunger (13).

11. The autoinjector (1) according to claim 10, wherein the third sensor includes an encoder wheel and an optoelectronic coupler.

12. The autoinjector (1) according to any of the preceding claims, further comprising:
a cap (7) releasably coupled to the case (2) and having a grip adapted to releasably engage a needle boot (6).

13. The autoinjector (1) according to claim 12, further comprising:
a fourth sensor adapted to generate a fourth signal when the cap (7) is coupled to the case (2).

14. The autoinjector (1) according to claim 13, wherein the controller changes a color or illumination sequence of the one or more light emitting elements based on the first signal, the second signal, the third signal or the fourth signal.

15. The autoinjector (1) according to claim 10, wherein the controller operates the releasable locking mechanism based on the first signal, the second signal, the third signal or the fourth signal.
